# EUROPEAN PATENT SPECIFICATION

(11) **EP 1 863 493 B1**
(45) Date of publication and mention of the grant of the patent: **06.06.2012**
(21) Application number: 06706151.5
(22) Date of filing: 28.02.2006
(51) Int. Cl.: A61K 31/05, A61K 31/573, A61K 31/11, A61K 31/19, A61K 45/06, A61K 36/738, A61K 36/752, A61P 17/00, A61P 17/06, A61P 17/08

(54) **Corticosteroid containing formulation for treating psoriasis, eczema, ulcer and burns.**
Kortikosteroide mit einer Formulierung zur Behandlung von Psoriasis, Ekzem, Geschwüren und Verbrennungen
Formulation contenant un corticostéroïde pour traiter le psoriasis, l'eczéma, l'ulcère et les brûlures.

(30) Priority: 01.03.2005 EG 20050102
(43) Date of publication of application: 12.12.2007
(73) Proprietor: El Masri, Mohamed Zakaria Ahmed, Alexandria (EG)
(72) Inventor: El Masri, Mohamed Zakaria Ahmed, Alexandria (EG)
(74) Representative: Chaillot, Geneviève
(86) International application number: PCT/EG2006/000009
(87) International publication number: WO 2006/092151

(56) References cited:
- WO-A-01/28491
- WO-A-2004/084855
- US-A1- 2003 229 141
- US-A1- 2005 014 827
- US-B1- 6 656 928
- STEVENSEN C J: "Aromatherapy in dermatology." CLINICS IN DERMATOLOGY 1998 NOV-DEC, vol. 16, no. 6, November 1998 (1998-11), pages 689-694, XP002529040 ISSN: 0738-081X
- "Psoriasis" In: Cassell, Dana ; Rose, Noel: "The Encyclopedia of autoimmune diseases", 2003, Facts on file, New York ISBN: 0-8160-4340-X page 182,
- "Allergy, Asthma and Immunology subspecialty consult" In: Barabara capest Jost, Elisabeth Friedman, Khaled M. Abdel-Hamid, Alpa L. Jani: "Washington Manual", 2003, Lippincott Williams & Wilkins, USA page 70,

## Description

### Technical field

Pharmaceutical preparation patent for dermatological use only

### Background art

### Topical treatment

Treatments applied directly to the skin are sometimes effective in clearing psoriasis.

Sunlight -daily- regular, short doses of sunlight without burning clears psoriasis in many people with the disease

### Phototherapy

UV light from the sun stimulates production of vitamin D by the skin which slows the overproduction of skin cells that causes scaling, daily , short , nonburning exposure to sunlight clears or improves psoriasis in some people therefore : sunlight may be included among initial treatments for the disease. A more controlled artificial light treatment may be used in mild psoriasis (UVB phototherapy or in more server or extensive psoriasis (psoralen and ultraviolet PUVA therapy).

**UVB phototherapy -** artificial sources of UVB light are similar to sunlight. some physicians will start with UVB treatments instead of topical agents.UVB phototherapy also is used to treat widespread psoriasis and lesions that resist topical treatment . This type of phototherapy is normally administered in a doctor's office by using a light panel or light box. Although with a doctor's guidance, some patients can use UVB light boxes at home.

**Corticosteroids-** available in different strengths, corticosteroids (cortisone) are usually applied twice each day. short-term treatment is often effective. if less than 10 percent of the body's skin is involved. some doctors will begin treatment with a high-potency corticosteroid ointment (for example, diprolene. temovate, ultravate or psorcon). high-potency steroids may also be used for treatment resistant plaques, particularly those on the hands or feet.

**Calcipotriene -** this drugs is a synthetic form of vitamins D3 (this is not the same as vitamins D supplements) application of calcipotriene ointment (for example, dovonex) twice daily controls the excessive production of skin cells in psoriasis

**Coal tar -** coal tar may be applied directly to the skin, used in a bath solution, or used as a shampoo for the scalp, it is available in different strengths

**Anthralin-**doctors sometimes use a 15- to 30- minute application of anthralin ointment, cream, or paste to treat chronic psoriasis lesions.

**Salicylic acid -** used to remove scales, salicylic acid is usually more effective when combined with topical steroids, anthralin , or coal tar.

**Bath solutions -** people with psoriasis may find that bathing in water with oil added then applying a moisturizer. can soothe the skin , scakes can be removed and itching reduced by soaking 15 minutes in water containing coal tar solution , oiled oatmeal , Epsom salts , or dead sea salts.

**Moisturizers:** when applied regularly over a long period. moisturizers have cosmetic and soothing effect. Preparations that are thick and greasy usually work best because they lock water into the skin

### Systemic treatment

Doctors sometimes prescribe medicines that are taken internally for more severe forms of psoriasis , particularly when more than 10 percent of the body is involved.

**Retinoids -** these drugs are derived from vitamin A and include etretinate (tegison) and isotretinoin (accutane) etretinate is most effective against pustular psoriasis.

**Methotrexate -** this treatment , which can be taken by pill or injection , **Hydroxyurea** (hydrea) - compared with methotrexate hydroxyurea is less toxic but also less effective hydroxyurea is sometimes combined PUVA or retinoids .

### Phototherapy

Sunlight may be included among initial treatment for the disease

### UVB phototherapy

It is associated with more side effects, including nausea , headache, fatigue , burning , and itching. long -term treatment is associated with irregular skin pigmentation.

### Corticosteroids

Long-term use or overuse of high-potency corticoSteroids can lead to thinning of skin , internal side effects , resistance to the treatment's benefits, and worsening of the psoriasis the international dose from the high- potency is 50 m/g for each 100g/m of ointment w/w for example (dermovite ointment or cream each 100g/m provides 50m/g high potency clobetasole propionate , each 100gm of elcon cream or ointment provides 100 mg high- potency corticosteroids w/w , each 100gm ointment of diprosalic provides 50m.g w/w of Beta methasone dipropionat (high-potency corticoSteroids)

### Calcipotriene -

The safety of using the drugs for psoriasis affecting more than 20 percent of the body's skin is unknown : use on widespread - area of skin may raise the amount of calcium in the body to unhealthy levels

### Coal tar :

Less effective and thus is not popular with many patients other drawbacks include its failure to provide long-term help for most patients , it's strong ouder , and its tendency to stain skin

### Anthralin

This treatment often fails to adequaltely clear lesions , it irritates the skin , and it stains skin and clothing brown or purple in additions unsuitable for acute or actively inflamed eruptions

### Salicylic acid :

Overuse cause salicyism

### Systemic treatment

### Retinoids :

And include etertinate (tegison) and isortetinoin (accutane)

Both drug can cause birth defects and are not recommende for women of childbearing age , at high doses , etretinete can effect liver function , therefore it is often combined with UVB phototherapy PUVA so that a lower , less toxic , does can be taken.

**Methotrexate -** this treatment , which can be taken by pill or injection , slows down cell production and suppresses the immune system patients taking methorexate must be closely carrying red blood cells , infection - fighting white blood cells , and clot-enhancing platelets as a precaution , doctors do not prescribe the drug for people with long term livers disease or anemia also , methotrexate should not be used by pregnant women , by women who are planning to get pregnant , or by their male partners.

US 6 656 928 discloses the use of corticosteroids in combination with a drying agent such as acetic acid and an anti-fungal agent such as thymol for the treatment of various skin disorders.

US 2003/229141 discloses the use of an anti-oxidant in combination with betamethasone dipropionate, clobetasol propionate, thymol or vitamins for the treatment of skin disorders.

WO 2004/084855 discloses the use of vanillin for the treatment of psoriasis.

STEVENSEN C. J. (CLINICS IN DERMATOLOGY vol. 16, no. 6, November 1998, pages 689-694) discloses the use of essential oils such as citrus oil and rose oil for the treatment of skin disrders including psoriasis.

WO 01/28491 discloses the use of essential oils such as rose oil for the treatment of psoriasis.

### Disclosure of invention

Composition: active ingredients, acetic acid , thymol , vanillin, high potency corticosteroid such as clobetasole propionate or betamethasone dipropionate, rose oil or lemon oil and antioxidant trace. High potency corticosteroids actions are anti inflammatory, anti pruritic. Acetic acid has keratolytic as well as bactericidal and fungicidal actions Vanillin has potent fungicidal and favouring agent . Thymol has antiseptic propriety. Rose oil or lemon oil has soothing effect for skin. The patent demonstrates these actions in a sustained manner thereby permitting twice a day application.

### Indications and usage :

The patent is indicated for the relief of the inflammatory manifestations of hyper keratolytic and dry corticosteroid - responsive dermatoses such as psoriasis , chronic a topic dermatitis , dermatitis such as lichen simplex chronicus and licken planus , eczema including nummular eczema , hard eczema , eczematous dermatitis , dyshidrosis, seborrhaic dermatitis of the scalp, ichthyosis vulgaris and other ichthyolic conditions. It is also indicated for relief of scaling and flaking associated with skin conditions. Also indicated for bed ulcer , leg ulcer , all forms of burns and ulcerations.

### Dosage and administration :

In form of ( ointment, cream, solution , lotion , emultion , spray) a thin film should be applied to cover completely the affected area twice daily in the morning and at night.

In form of (spray) shaken it before use and spray just to cover the affective area with a fine coat products twice daily.

The patent provides in each 100 gram of (ointment , cream , solution , lotion, emultion, spray) from 2mg to 7 mg w\w high potency corticosteroid consequently adverse reactions that have been reported with the use of topical corticosteroids (include burning , itching, irritation, dryness , eruptions , hypopigmentation, dermatitis) completely disappear and therefore must be born in mind when long-term continous therapy is very safe. Where no possible particularly in infants and children as adrenal suppression can occur. Use this patent for treating such conditions as psoriasis , discoid lupus erythematosus and sever eczema is also very safe.

## Claims

1. A pharmaceutical composition comprising, as active ingredients, acetic acid, thymol, vanillin, a high potency corticosteroid, rose oil or lemon oil and trace of anti-oxidant, wherein the high potency corticosteroid is clobetasole propionate or betamethasone dipropionate.

2. The pharmaceutical composition according to claim 1, wherein acetic acid is 96 % acetic acid present from 0,5 g to 2 g in each 100 g of said pharmaceutical composition.

3. The pharmaceutical composition according to anyone of claims 1 or 2, wherein thymol is present from 25 mg to 150 mg in each 100 g of said pharmaceutical composition.

4. The pharmaceutical composition according to anyone of claims 1 to 3, wherein vanillin is present from 0,5 g to 3 g in each 100 g of said pharmaceutical composition.

5. The pharmaceutical composition according to anyone of claims 1 to 4, wherein the high potency corticosteroid is present from 2 mg to 7 mg in each 100 g of said composition.

6. The pharmaceutical composition according to anyone of claims 1 to 5, wherein rose oil or lemon oil is present from 0,5 g to 4 g in each 100 g of said pharmaceutical composition.

7. The pharmaceutical composition according to anyone of claims 1 to 6 containing vitamins.

8. The pharmaceutical composition according to anyone of claims 1 to 7, which is in the form of a cream, an ointment, an emulsion, a lotion, a solution or a spray.

9. Use of the pharmaceutical composition as defined in anyone of claims 1 to 8 for the manufacture of a medicament for the relief of the inflammatory manifestations of hyperkeratolytic and dry corticosteroid-responsive dermatoses such as psoriasis, chronic atopic dermatitis, dermatitis such as lichen simplex chronicus and lichen planus, eczema such as nummular eczema, hard eczema, eczematous dermatitis, dyshidrosis, seborrheaic dermatitis of the scalp, ichthyosis vulgaris and other ichthyolic conditions, scaling and flaking associated with skin conditions, bed ulcer, leg ulcer, and all forms of burns and ulcerations.

## Patentansprüche

1. Pharmazeutische Zusammensetzung, umfassend, als aktive Bestandteile, Essigsäure, Thymol, Vanillin, ein hochwirksames Corticosteroid, Rosenöl oder Zitronenöl und Spuren eines Antioxidationsmittels, wobei das hochwirksame Corticosteroid Clobetasolpropionat oder Betamethasondipropionat ist.

2. Pharmazeutische Zusammensetzung nach Anspruch 1, wobei Essigsäure 96%-ige Essigsäure ist, die in je 100 g der pharmazeutischen Zusammensetzung von 0,5 g bis 2 g vorhanden ist.

3. Pharmazeutische Zusammensetzung nach einem der Ansprüche 1 oder 2, wobei Thymol in je 100 g der pharmazeutischen Zusammensetzung von 25 mg bis 150 mg vorhanden ist.

4. Pharmazeutische Zusammensetzung nach einem der Ansprüche 1 bis 3, wobei Vanillin in je 100 g der pharmazeutischen Zusammensetzung von 0,5 g bis 3 g vorhanden ist.

5. Pharmazeutische Zusammensetzung nach einem der Ansprüche 1 bis 4, wobei das hochwirksame Corticosteroid in je 100 g der Zusammensetzung von 2 mg bis 7 mg vorhanden ist.

6. Pharmazeutische Zusammensetzung nach einem der Ansprüche 1 bis 5, wobei Rosenöl oder Zitronenöl in je 100 g der pharmazeutischen Zusammensetzung von 0,5 g bis 4 g vorhanden ist.

7. Pharmazeutische Zusammensetzung nach einem der Ansprüche 1 bis 6, die Vitamine enthält.

8. Pharmazeutische Zusammensetzung nach einem der Ansprüche 1 bis 7, die in Form einer Creme, einer Salbe, einer Emulsion, einer Lotion, einer Lösung oder eines Sprays vorliegt.

9. Verwendung der pharmazeutischen Zusammensetzung gemäß Definition in einem der Ansprüche 1 bis 8 zur Herstellung eines Medikaments zur Linderung der Entzündungserscheinungen von hyperkeratolytischen und trockenen auf Corticosteroid ansprechenden Dermatosen wie Psoriasis, chronische atopische Dermatitis, Dermatitis wie Lichen simplex chronicus und Lichen ruber planus, Ekzeme wie nummuläres Ekzem, schweres Ekzem, ekzematöse Dermatitis, Dyshidrosis, seborrhoische Dermatitis der Kopfhaut, Ichthyosis vulgaris und weitere ichthyotische Erkrankungen, Schuppung und Abschilferung im Zusammenhang mit Hauterkrankungen, Druckgeschwür, Beingeschwür und sämtliche Formen von Verbrennungen und Geschwüren.

## Revendications

1. Composition pharmaceutique comprenant, en tant que principes actifs, de l'acide acétique, du thymol, de la vanilline, un corticostéroïde à puissance élevée, de l'huile de rose ou de l'huile de citron, et des traces d'antioxydant, dans laquelle le corticostéroïde à puissance élevée est du propionate de clobétasol ou du dipropionate de bêtaméthasone.

2. Composition pharmaceutique selon la revendication 1, dans laquelle l'acide acétique est de l'acide acétique à 96 % présent de 0,5 g à 2 g pour 100 g de ladite composition pharmaceutique.

3. Composition pharmaceutique selon l'une quelconque des revendications 1 ou 2, dans laquelle le thymol est présent de 25 mg à 150 mg pour 100 g de ladite composition pharmaceutique.

4. Composition pharmaceutique selon l'une quelconque des revendications 1 à 3, dans laquelle la vanilline est présente de 0,5 g à 3 g pour 100 g de ladite composition pharmaceutique.

5. Composition pharmaceutique selon l'une quelconque des revendications 1 à 4, dans laquelle le corticostéroïde à puissance élevée est présent de 2 mg à 7 mg pour 100 g de ladite composition.

6. Composition selon l'une quelconque des revendications 1 à 5, dans laquelle l'huile de rose ou l'huile de citron est présente de 0,5 g à 4 g pour 100g de ladite composition pharmaceutique.

7. Composition pharmaceutique selon l'une quelconque des revendications 1 à 6, contenant des vitamines.

8. Composition pharmaceutique selon l'une quelconque des revendications 1 à 7, qui est sous la forme d'une crème, d'un onguent, d'une émulsion, d'une lotion, d'une solution ou d'une pulvérisation.

9. Utilisation de la composition pharmaceutique telle que définie à l'une quelconque des revendications 1 à 8, pour la fabrication d'un médicament destiné à le soulagement des manifestations inflammatoires de dermatose hyperkératolytique et de dermatose sèche sensible aux corticostéroïdes, telles que le psoriasis, la dermatite chronique atopique, la dermatite telle que le lichen simplex chronique et le lichen plan, l'eczéma tel que l'eczéma nummulaire, l'eczéma sévère, la dermatite eczémateuse, la dyshidrose, la dermatite séborrhéique du cuir chevelu, l'ichthyose vulgaire et d'autres états ichthyoliques, une desquamation et un effritement associés à des états cutanés, un ulcère de pression, un ulcère de la jambe, et toutes formes de brûlures et d'ulcères.
